# EUROPEAN PATENT APPLICATION

(11) **EP 3 087 975 A1**
(43) Date of publication of application: **02.11.2016**
(21) Application number: 16169370.0
(22) Date of filing: 26.08.2005
(51) Int. Cl.: A61K 9/16, A61K 9/20

(54) **TREATMENT WITH VILDAGLIPTIN**

(30) Priority: 27.08.2004 US 605327 P; 07.10.2004 US 616828 P
(62) Divisional of application: 05775067.1
(71) Applicant: NOVARTIS AG, 4056 Basel (CH); Novartis Pharma GmbH, 1230 Wien (AT)
(72) Inventor: KOWALSKI, James, Belle Mead, NJ 08502 (US); KALB, Oskar, 79539 Lörrach (DE); SERAJUDDIN, Abu, T., M., Queens, NY 11358 (US); JOSHI, Yatindra, Princeton, NJ 08540 (US)
(74) Representative: Hutchison, John Robert

(57) **Abstract**

DPP-IV inhibitor compound for use in treating non-insulin-dependent diabetes mellitus, wherein the compound is (S)-1-[(3-hydroxy-1-admantyl)amino]acetyl-2-cyano-pyrrolidine of formula (IIC) 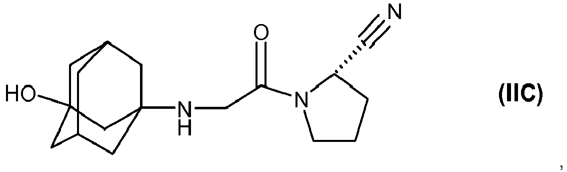 and optionally pharmaceutical salts thereof, wherein the dosage of the compound of formula (IIC) is between 50 and 100 mg per day and the application of the compound of formula (IIC) occurs twice a day.

## Description

### Field of the Invention

The present invention relates to immediate-release pharmaceutical compositions comprising a moisture-sensitive therapeutic compound and a hydrophobic melt component. The present invention also relates to processes for making such immediate-release pharmaceutical compositions.

### Background of the Invention

A factor that can limit the commercial viability of a therapeutic compound is that compound's susceptibility to moisture. Moisture-sensitive therapeutic compounds may be difficult to formulate into pharmaceutically acceptable oral compositions because of their chemical instability. This chemical instability may particularly manifest when the moisture-sensitive therapeutic compound is used in a dosage form containing an excipient with a high equilibrium moisture content. Moisture can migrate from such excipients and cause the moisture-sensitive therapeutic compound to undergo hydrolytic degradation.

A process used to minimize the chemical instability of a moisture therapeutic compound is melt granulation, also known as thermal infusion. Melt granulation is a process which involves the use of solid and/or semi-solid materials that are mixed and heated with the therapeutic compound to form granules of the solid and/or semi-solid material coated on particulates of the therapeutic compound.

However, melt granulation is also used as a method for manufacturing extended-release or controlled-release pharmaceutical compositions. There exists a need for a pharmaceutical composition that incorporates melt granulated granules while still retaining immediate-release characteristics. The present invention addresses this need.

### Summary of the Invention

The present invention relates to a pharmaceutical composition for the oral administration of a moisture-sensitive therapeutic compound. The composition includes melt granules of the moisture-sensitive therapeutic compound and a hydrophobic melt component. Such pharmaceutical compositions provide for better chemical stability of the therapeutic compound especially in the presence of moisture or water. Additionally, the pharmaceutical compositions of the present invention are immediate-release compositions that do not have the characteristics or profiles of extended-release or controlled-release formulations.

In another aspect of the present invention, a process for making melt granules of a therapeutic compound and a hydrophobic melt component is taught. The process generally includes the following steps:
(a) forming a mixture of a moisture-sensitive therapeutic compound with at least one hydrophobic melt component;
(b) heating said mixture to a temperature above, near, substantially near, or at the melting range of said hydrophobic melt component;
(c) granulating the mixture under high shear to form the granules; and
(d) cooling the granules to room temperature.

In yet another aspect of the present invention, the pharmaceutical composition comprises particles of a moisture-sensitive therapeutic compound coated or substantially coated with a hydrophobic melt component. The moisture-sensitive therapeutic compound is, for example, a DPP-IV inhibitor.

### Detailed Description of the Invention

The present invention relates to a process for preparing immediate-release solid dosage forms of a moisture-sensitive therapeutic compound which comprises melt granulated granules of the moisture-sensitive therapeutic compound with a hydrophobic melt component.

As used herein the term "pharmaceutical composition" means a mixture or solution containing a moisture-sensitive therapeutic compound to be administered to a mammal, e.g., a human in order to prevent, treat or control a particular disease or condition affecting the mammal.

As used herein the term "therapeutic compound" means any compound, substance, drug, medicament, or active ingredient having a therapeutic or pharmacological effect, and which is suitable for administration to a mammal, e.g., a human, in a composition that is particularly suitable for oral administration.

Examples of therapeutic classes of therapeutic compounds include, but are not limited to, antihypertensives, antianxiety agents, anticlotting agents, anticonvulsants, blood glucose-lowering agents, decongestants, antihistamines, antitussives, antineoplastics, beta blockers, anti-inflammatories, antipsychotic agents, cognitive enhancers, anti-atherosclerotic agents, cholesterol reducing agents, antiobesity agents, autoimmune disorder agents, anti-impotence agents, antibacterial and antifungal agents, hypnotic agents, antibiotics, antidepressants, antiviral agents and combinations of the foregoing.

The therapeutic compound(s) is present in the pharmaceutical compositions of the present invention in a therapeutically effective amount or concentration. Such a therapeutically effective amount or concentration is known to one of ordinary skill in the art as the amount or concentration varies with the therapeutic compound being used and the indication which is being addressed. For example, in accordance with the present invention, the therapeutic compound may be present in an amount by weight of up to about 20% by weight of the pharmaceutical composition, e.g., from about 0.05% by weight. The therapeutic compound may also be present in an amount from about 0.5% to 15% by weight of the pharmaceutical composition, e.g., from about 1.5% to about 5% by weight of the pharmaceutical composition.

As used herein, the term "moisture-sensitive therapeutic compound" refers to a therapeutic compound which undergoes spontaneous degradation, e.g., by hydrolysis of at least 1% by weight of the therapeutic compound when the therapeutic compound contacts water. Thus, moisture-sensitive therapeutic compounds often are not formulated to be in direct contact with excipients that have a high, or appreciable, equilibrium moisture content. As used herein, an excipient with a high, or appreciable, equilibrium moisture content is one with greater than 5% moisture content at 25°C and 75% relative humidity (RH). For example, such excipients include, but are not limited to, microcrystalline cellulose, pregelatinized starch, corn starch and povidone.

Therapeutic compounds of particular interest for use in the present invention are dipeptidylpeptidase IV (DPP-IV) inhibitors especially the *N*-(substituted glycyl)-2-cyanopyrrolidones, such as those described in U.S. Patent Nos. 6,011,155 and 6,166,063 (the '063 patent), which are hereby incorporated by reference. These compounds and their corresponding pharmaceutically acceptable acid addition salts are useful in the treatment of conditions, such as non-insulin-dependent diabetes mellitus, arthritis, obesity, allograft transplantation and calcitonin-osteoporosis. In addition, based on the roles of glucagon-like peptides, such as GLP-1 and GLP-2, and their association with DPP-IV inhibition, it is expected that the compounds disclosed herein are useful, e.g., to produce a sedative or anxiolytic effect, or to attenuate post-surgical catabolic changes and hormonal responses to stress, or to reduce mortality and morbidity after myocardial infarction, or in the treatment of conditions related to the above effects which may be mediated by GLP-1 and/or GLP-2 levels.

More specifically, e.g., the DPP-IV inhibitor compounds disclosed herein and their corresponding pharmaceutically acceptable addition salts improve early insulin response to an oral glucose challenge and, thus, are useful in treating non-insulin-dependent diabetes mellitus.

An example of such a compound is [S]-1-[2-(5-cyano-2-pyridinylamino) ethylamino]acetyl-2-pyrolidine carbonitrile monohydrochloride as shown in formula (I)

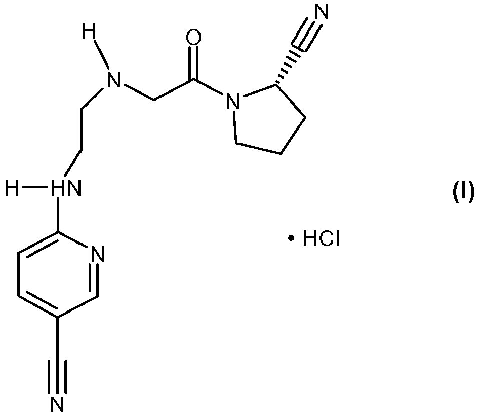

This monohydrochloride of formula (I) (hereinafter, "Compound I") is described in scheme 2 of Villhauer et al., J Med Chem, Vol. 45, No. 12, pp. 2362-65 (2002), which is hereby incorporated by reference. Compound I is a moisture-sensitive therapeutic compound. At humidities above 75%, Compound I absorbs moisture and begins to deliquesce; moreover, Compound I undergoes both acidic and basic degradation with cyclic imidate ("CI") being the major degradation product.

Another example of *N*-(substituted glycyl)-2-cyanopyrrolidines useful in the present invention are those of the following formula (II) 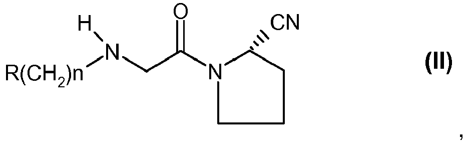 wherein
R is substituted adamantyl; and
n is 0-3;
in free form or in acid addition salt form.

The term "substituted adamantyl", as used herein, refers to adamantyl, i.e., 1- or 2-adamantyl, substituted by one or more, e.g., two substituents selected from alkyl, -OR₁ or -NR₂R₃, where R₁, R₂ and R₃ are, independently, hydrogen, alkyl, C₁-C₈alkanoyl, carbamyl, or -CO-NR₄R₅, where R₄ and R₅ are, independently, alkyl, unsubstituted or substituted aryl and where one of R₄ and R₅ additionally is hydrogen or R₄ and R₅ together represent C₂-C₇alkylene.

The term "aryl", as used herein, represents phenyl. Substituted phenyl particularly is phenyl substituted by one or more, e.g., two, substitutents selected from, e.g., alkyl, alkoxy, halogen and trifluoromethyl.

The term "alkoxy", as used herein, refers to alkyl-O-.

The term "halogen" or "halo", as used herein, refers to fluorine, chlorine, bromine and iodine.

The term "alkylene", as used herein, refers to a straight chain bridge of 2-7 carbon atoms, e.g., 3-6 carbons, or yet another example, 5 carbon atoms.

A particular group of compounds useful in the present invention is the compounds of formula (II), wherein the substituent on the adamantyl is bonded on a bridgehead or a methylene adjacent to a bridgehead. Compounds of formula (II), wherein the glycyl-2-cyanopyrrolidine moiety is bonded to a bridgehead, the R' substituent on the adamantyl is particularly 3-hydroxy. Compounds of formula (II), wherein the glycyl-2-cyanopyrrolidine moiety is bonded at a methylene adjacent to a bridgehead, the R' substituent on the adamantyl is particularly 5-hydroxy.

The present invention especially relates to a compound of formula (IIA) (hereinafter "Compound IIA") or formula (IIB) (hereinafter "Compound IIB") 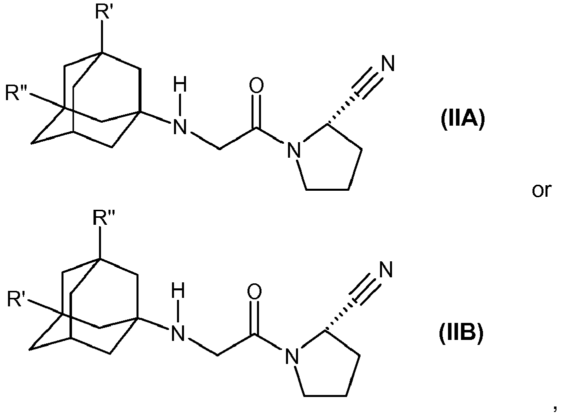 wherein
R' represents hydroxy, C₁-C₇alkoxy, C₁-C₈alkanoyloxy or R₅R₄N-CO-O-, where R₄ and R₅ independently are C₁-C₇alkyl or phenyl which is unsubstituted or substituted by a substituent selected from C₁-C₇alkyl, C₁-C₇alkoxy, halogen and trifluoromethyl and where R₄ additionally is hydrogen; or R₄ and R₅ together represent C₃-C₆alkylene; and
R" represents hydrogen; or
R' and R", independently, represent C₁-C₇alkyl,
in free form or in form of a pharmaceutically acceptable acid addition salt.

These DPP-IV inhibitor compounds of formula (II), (IIA) or (IIB) are known and described in the '063 patent. They can exist in free form or in acid addition salt form. Pharmaceutically acceptable, i.e., non-toxic and physiologically acceptable, salts are especially useful, although other salts are also useful, e.g., in isolating or purifying the compounds of this invention. Acid addition salts include the hydrochlorides, salts of methanesulfonic, sulfuric, phosphoric, citric, lactic and acetic acid may also be utilized.

A particularly useful compound of formula (II), (IIA) or (IIB) is (*S*)-1-[(3-hydroxy-1-admantyl)amino]acetyl-2-cyano-pyrrolidine of formula (hereinafter "Compound IIC") 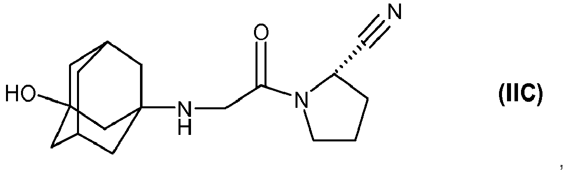 and optionally pharmaceutical salts thereof.

The dosage of Compound IIC, e.g., is between about 10 mg and about 150 mg per day; e.g., between about 24 mg and 100 mg per day; e.g., between about 50 mg and 100 mg per day. Examples of daily oral dosage are 25, 30, 35, 45, 50, 55, 60, 80 or 100 mg. The application of the therapeutic compound may occur up to three times a day, or once or twice a day.

Also useful DPP-IV inhibitors are those included in the specific examples of U.S. Patent Nos. 6,124,305 and 6,107,317; PCT Publication Nos. WO 98/19998, WO 95/15309 and WO 98/18763, which are hereby incorporated by reference. Among these examples are 1 [2-[(5-cyanopyridin-2-yl)aminoethylamino]acetyl-2-cyano-(S)-pyrrolidine and (2*S*)-I-[(2*S*)-2 amino-3,3-dimethylbutanoyl]-2-pyrrolidinecarbonitrile.

In yet another embodiment, the DPP-IV inhibitor useful in the present invention is a *N*-peptidyl-*O*-aroyl hydroxylamine or a pharmaceutically acceptable salt thereof. Aroyl is, e.g., naphthylcarbonyl; or benzoyl which is unsubstituted or mono- or disubstituted, e.g., by lower alkoxy, lower alkyl, halogen or preferably, nitro. The peptidyl moiety comprises preferably two α-amino acids, e.g., glycine, alanine, leucine, phenylalanine, lysine or proline, of which the one attached directly to the hydroxylamine nitrogen atom is particularly proline.

For example, the *N*-peptidyl-*O*-aroyl hydroxylamine is a compound of formula (IV) 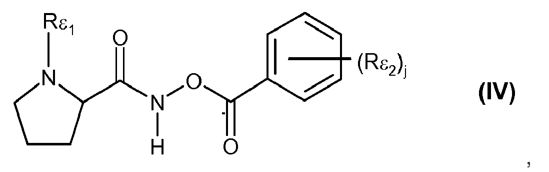 wherein
j is 0, 1 or 2;
Rε₁ represents the side chain of a natural amino acid; and
Rε₂ represents lower alkoxy, lower alkyl, halogen or nitro,
or a pharmaceutically acceptable salt thereof.

In a particular embodiment of the invention, the *N*-peptidyl-*O*-aroyl hydroxylamine is a compound of formula (IVA) 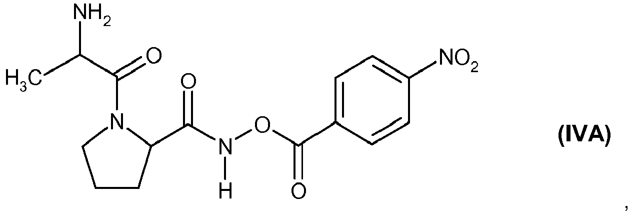 or a pharmaceutically acceptable salt thereof.

*N*-Peptidyl-O-aroyl hydroxylamines, e.g., of formula (IV) or (IVA), and their preparation are described by Demuth et al., J Enzyme Inhibition, Vol. 2, pp. 192-142 (1988), which is hereby incorporated by reference.

In further embodiments, useful DPP-IV inhibitors are *N*-substituted adamantyl-amino-acetyl-2-cyano pyrrolidines, *N*-(substituted glycyl)-4-cyano pyrrolidines, *N*-(*N*'-substituted glycyl)-2-cyanopyrrolidines, *N*-aminoacyl thiazolidines, *N*-aminoacyl pyrrolidines, *L*-allo-isoleucyl thiazolidine, L-threo-isoleucyl pyrrolidine and *L*-allo-isoleucyl pyrrolidine, 1-[2-[(5-cyanopyridin-2-yl)amino]ethylamino]acetyl-2-cyano-(S)-pyrrolidine and pharmaceutical salts thereof.

Additional DPP-IV inhibitors useful in the present invention are also those described by Patel et al., Exp Opin Invest Drugs, Vol. 12, No. 4, pp. 623-33 (2003) in paragraph 5, especially P32/98, K-364, FE-999011, BDPX, NVP-DDP-728 and others, which publication is hereby incorporated by reference.

FE-999011 is also described in the PCT Publication No. WO 95/15309 as compound No. 18.

P32/98 or P3298 (CAS number: 251572-86-8) also known as 3-[(2S,3S)-2-amino-3-methyl-1-oxopentyl]thiazolidine can be used as 3-[(2S,3S)-2-amino-3-methyl-1-oxopentyl]thiazolidine and (2*E*)-2-butenedioate (2:1) mixture, such as shown below: 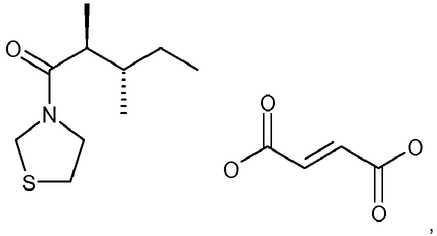 and is described in PCT Publication No. WO 99/61431 in the name of Probiodrug.

Additional DPP-IV inhibitors useful in the present invention are described in the PCT Publication Nos. WO 04/037169, Examples 1-48; WO 02/062764, Examples 1-293; and WO 04/024184, which are all hereby incorporated by reference. Examples in these publications include 3-(aminomethyl)-2-isobuthyl-1-oxo-4-phenyl-1,2-dihydro-6-isoquinolinecarboxamide and 2-{[3-(aminomethyl)-2-isobuthyl-4-phenyl-1-oxo-1,2-dihydro-6-isoquinolyl]oxy}acetamide described on Page 7.

PCT Publication No. WO 03/004498 discloses other DPP-IV inhibitors in Examples 1-33. A particular compound of formula (V), Example 7 and known as MK-0431, disclosed therein is:

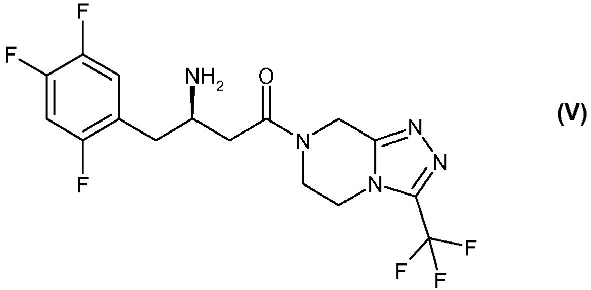

DPP-IV inhibitors useful in the present invention are also described by Ashton, Bioorg Med Chem Lett, Vol. 14, pp. 859-63 (2004), especially the Compound 1 or the Compound 21e (see Table 1) and the compounds listed in the Tables 1 and 2. Useful DPP-IV inhibitors are also described in the PCT Publication No. WO 04/037181, which is hereby incorporated by reference, especially Examples 1-33 and most preferably the compounds described in the Claims 3-5.

As used herein, the term "immediate-release" refers to the rapid release of the majority of the therapeutic compound, e.g., greater than about 50%, about 60%, about 70%, about 80%, or about 90% within a relatively short time, e.g., within 1 hour, 40 minutes, 30 minutes or 20 minutes after oral ingestion. Particularly useful conditions for immediate-release are release of at least or equal to about 80% of the therapeutic compound within thirty minutes after oral ingestion. The particular immediate-release conditions for a specific therapeutic compound will be recognized or known by one of ordinary skill in the art.

As used herein, the term "controlled-release" refers to the gradual but continuous or sustained release over a relatively extended period of the therapeutic compound content after oral ingestion and which starts when the pharmaceutical composition reaches the stomach and starts to disintegrate. The release will continue over a period of time and may continue through until and after the pharmaceutical composition reaches the intestine. Controlled-release may also refer to delayed-release in which release of the therapeutic compound does not start immediately when the pharmaceutical composition reaches the stomach but is delayed for a period of time, for instance, until when the pharmaceutical composition reaches the intestine when the increasing pH is used to trigger release of the therapeutic compound from the pharmaceutical composition.

As used herein, the term "hydrophobic", with respect to the melt component, refers to being more compatible with oil than with water. A substance with hydrophobic properties is insoluble or almost insoluble in water but are easily soluble in oil or other nonpolar solvents.

As used herein the term "melt component" refers to substances or a mixtures of substances that are solid or semi-solid at room temperature (about 25°C) and with relatively low melting temperatures, e.g., from about 30°C to about 100°C, or from about 50°C to about 80°C. Such melt components when heated to a temperature at or near their melting ranges will transition from a solid phase to a liquid phase. As used herein, the term "melting range" refers to the range of temperatures from the lower temperature at which the first drop of liquid begins to form from the solid phase to the higher temperature at which the entire mass of solid material becomes a liquid material.

Examples of hydrophobic melt components include, but are not limited to, esters, hydrogenated oils, natural waxes, synthetic waxes, hydrocarbons, fatty alcohols, fatty acids, monoglycerides, diglycerides, triglycerides and mixtures thereof. Examples of esters, such as glyceryl esters include, but are not limited to, glyceryl monostearate, e.g., CAPMUL GMS from Abitec Corp. (Columbus, OH); glyceryl palmitostearate; acetylated glycerol monostearate; sorbitan monostearate, e.g., ARLACEL 60 from Uniqema (New Castle, DE); and cetyl palmitate, e.g., CUTINA CP from Cognis Corp. (Düsseldorf, Germany). Examples of hydrogenated oils include, but are not limited to, hydrogenated castor oil, e.g., CUTINA HR from Cognis Corp; hydrogenated cottonseed oil; hydrogenated soybean oil; and hydrogenated palm oil. Examples of waxes include, but are not limited to, carnauba wax, beeswax and spermaceti wax. Examples of hydrocarbons include, but are not limited to, microcrystalline wax and paraffin. Examples of fatty alcohols, i.e., higher molecular weight nonvolatile alcohols that have from about 14 to about 31 carbon atoms include, but are not limited to, cetyl alcohol, e.g., CRODACOL C-70 from Croda Corp. (Edison, NJ) ; stearyl alcohol, e.g., CRODACOL S-95 from Croda Corp; lauryl alcohol; and myristyl alcohol. Examples of fatty acids which may have from about 10 to about 22 carbon atoms include, but are not limited to, stearic acid, e.g., HYSTRENE 5016 from Crompton Corp. (Middlebury, CT); decanoic acid; palmitic acid; lauric acid; and myristic acid.

The hydrophobic melt component, for example, may be present in an amount from about 1 % to about 70% by weight of the pharmaceutical composition, e.g., from about 10% to about 60%, e.g., from about 20% to about 40%.

As used herein, the term "melt granulation" refers to the general process that comprises the steps of:
(a) forming a mixture of a moisture-sensitive therapeutic compound with at least one hydrophobic melt component;
(b) heating the mixture to a temperature above, near, substantially near, i.e., within 10 °C, above, or at the melting range of hydrophobic melt component while the mixture is being mixed under high shear to form granules; and
(c) cooling the granules to room temperature at a controlled rate.

The heating and mixing of the moisture-sensitive therapeutic compound and the hydrophobic melt component to form melt granulated granules may be accomplished, e.g., by the use of fluidized bed granulator or a vessel supplied with high-shear mixing means. The hydrophobic component, e.g., may be present in an amount from about 40% to about 95% by weight of the composition of the granule, e.g., from about 50% to about 80%, e.g., from about 60% to about 75%. Similarly, the moisture-sensitive therapeutic compound, may be present in an amount from about 5% to about 60% by weight of the composition of the melt granulated granule, e.g., from about 20% to about 50%, e.g., from about 35% to about 40%.

The resulting melt granulated granules are, for example, particles of the moisture-sensitive therapeutic compound coated or substantially coated (e.g., at least 90% of the surface area) with the hydrophobic melt component. The coating may be, e.g., a continuous or discontinuous, regular or irregular, and uniform or non-uniform barrier around particles of the therapeutic compound.

Alternatively, in another aspect of the present invention, the resulting melt granulated granules are particles of the moisture therapeutic compound embedded or substantially embedded with or within the hydrophobic melt component. Such embedded granules of therapeutic compound can co-exist with other particles of the moisture therapeutic compound coated with the hydrophobic melt component.

Examples of high-shear mixing means includes, but is not limited to, a high-shear mixer, high-speed mixer, high-speed granulator or melt extruder. The melt granulation process is explained in more detail below.

Once the melt granulated granules are obtained, the granules may be formulated into oral forms, e.g., solid oral dosage forms, such as tablets, pills, lozenges, caplets, capsules or sachets. Such oral dosage forms may comprise conventional excipients used for pharmaceuticals. Examples of such excipients include, but are not limited to, disintegrants, binders, lubricants, glidants, stabilizers, fillers and diluents. One of ordinary skill in the art may select one or more of the aforementioned excipients with respect to the particular desired properties of the solid oral dosage form by routine experimentation and without any undue burden. The amount of each excipient used may vary within ranges conventional in the art. The following references which are all hereby incorporated by reference discloses techniques and excipients used to formulate oral dosage forms. See The Handbook of Pharmaceutical Excipients, 4th edition, Rowe et al., Eds., American Pharmaceuticals Association (2003); and Remington: the Science and Practice of Pharmacy, 20th edition, Gennaro, Ed., Lippincott Williams & Wilkins (2003).

Examples of pharmaceutically acceptable disintegrants include, but are not limited to, starches; clays; celluloses; alginates; gums; cross-linked polymers, e.g., cross-linked polyvinyl pyrrolidone or crospovidone, e.g., POLYPLASDONE XL from International Specialty Products (Wayne, NJ); cross-linked sodium carboxymethylcellulose or croscarmellose sodium, e.g., AC-DI-SOL from FMC; and cross-linked calcium carboxymethylcellulose; soy polysaccharides; and guar gum. The disintegrant, e.g., may be present in an amount from about 1% to about 20%, e.g., from about 5% to about 10%, e.g., about 5% about by weight of the composition.

Examples of pharmaceutically acceptable binders include, but are not limited to, starches; celluloses and derivatives thereof, for example, microcrystalline cellulose, e.g., AVICEL PH from FMC (Philadelphia, PA), hydroxypropyl cellulose hydroxylethyl cellulose and hydroxylpropylmethyl cellulose METHOCEL from Dow Chemical Corp. (Midland, MI); sucrose; dextrose; corn syrup; polysaccharides; and gelatin. The binder, e.g., may be present in an amount from about 5% to about 50%, e.g., 10-40% by weight of the composition.

Examples of pharmaceutically acceptable lubricants and pharmaceutically acceptable glidants include, but are not limited to, colloidal silica, magnesium trisilicate, starches, talc, tribasic calcium phosphate, magnesium stearate, aluminum sterate, calcium stearate, magnesium carbonate, magnesium oxide, polyethylene glycol, powdered cellulose and microcrystalline cellulose. The lubricant, e.g., may be present in an amount from about 0.1 % to about 5% by weight of the composition; whereas, the glidant, e.g., may be present in an amount from about 0.1 % to about 10% by weight.

Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. The filler and/or diluent, e.g., may be present in an amount from about 15% to about 40% by weight of the composition.

To make immediate-release solid oral dosage forms prepared from melt granulated granules begins with obtaining a moisture-sensitive therapeutic compound and a hydrophobic melt component that has each been screened, together or separately, through a sieve resulting in a maximum particle size of each component. One of ordinary skill in the art will appreciate the necessary particle size of each component that is necessary for the particular pharmaceutical composition being formulated. For example, suitable particle sizes, include those of less than equal to 1,000 µm, 750 µm, 500 µm or 250 µm.

The moisture-sensitive therapeutic compound and the hydrophobic melt component are mixed in a ratio of moisture therapeutic compound to hydrophobic melt component in a range of 1:0.25 to 1:1 to 1:10 (on a dry weight basis), or more particularly in a range of 1:1 to 1:4 (on a dry weight basis) in a vessel, e.g., a jacketed kettle, with high-shear mixing means. The mixture is heated, e.g., by steam in the jacket, to a temperature near, substantially near, at or above the melting range of the hydrophobic melt component. The mixture is maintained at the elevated temperature and blended for a time sufficient to form a substantially homogeneous granulated product. After a substantially homogeneous granulated product is obtained, the granulated mixture is cooled, e.g., by using cold water in the jacket, until solidified. The solidified granulated mixture may be continually cooled in the jacketed vessel to room temperature or be immediately removed from the jacketed vessel and cooled on aluminum foil (to allow slow cooling).

After cooling, the granules may be milled and subsequently screened through a sieve. The granules are then combined with solid oral dosage form excipients, i.e., fillers, binders, disintegrants and lubricants. The combined mixture may be further blended, e.g., through a V-blender, and subsequently compressed or molded into a tablet or encapsulated by a capsule.

The utility of all the pharmaceutical compositions of the present invention may be observed in standard clinical tests in, for example, known indications of drug dosages giving therapeutically effective blood levels of the therapeutic compound; for example using dosages in the range of 2.5-1000 mg of therapeutic compound per day for a 75 kg mammal, e.g., adult and in standard animal models.

The pharmaceutical composition, e.g., in form of a tablet or a powder suitable for tablet formulation will suitably contain between 0.1 mg and 100 mg of the therapeutic compound, e.g., 0.1, 1, 5, 10, 20, 25, 50 or 100 mg. Such unit dosage forms are suitable for administration one to five times daily depending upon the particular purpose of therapy, the phase of therapy and the like.

The present invention provides a method of treatment of a subject suffering from a disease, condition or disorder treatable with a moisture sensitive therapeutic compound comprising administering a therapeutically effective amount of a pharmaceutical composition of the present invention to a subject in need of such treatment. Additionally, the present invention provides the use of a composition according to the present invention comprising a DPP-IV inhibitor in the manufacture of a medicament for the treatment and/or prevention of conditions, such as non-insulin-dependent diabetes mellitus, arthritis, obesity, allograft transplantation and calcitonin osteoporosis, or a condition which may be mediated by GLP-1 and/or GLP-2 levels.

### The present invention comprises the following aspects:

1. A pharmaceutical composition for the oral administration of a moisture-sensitive therapeutic compound comprising granules comprising said moisture-sensitive therapeutic compound coated or substantially coated with a hydrophobic melt component, wherein said pharmaceutical composition is an immediate-release composition.
2. The pharmaceutical composition of aspect 1, wherein said granules are obtained by a melt granulation process.
3. The pharmaceutical composition of aspect 1, wherein said pharmaceutical composition releases at least about 50% of said moisture-sensitive therapeutic compound within 30 minutes after oral ingestion.
4. The pharmaceutical composition of aspect 3, wherein said pharmaceutical composition releases at least about 80% of said moisture-sensitive therapeutic compound within 30 minutes after oral ingestion.
5. The pharmaceutical composition of aspect 1, wherein said granules have a ratio of said moisture-sensitive therapeutic compound: said hydrophobic melt component in a range from 1:1 to 1:10.
6. The pharmaceutical composition of aspect 5, wherein said range is from 1:1 to 1:4.
7. The pharmaceutical composition of aspect 1, wherein said pharmaceutical composition further comprises a disintegrant in a range of from about 1% to 20% by weight of said composition.
8. The pharmaceutical composition of aspect 1, wherein said moisture-sensitive therapeutic compound is a *N*-(substituted glycyl)-2-cyanopyrrolidine or a pharmaceutically acceptable salt thereof.
9. The pharmaceutical composition of aspect 8, wherein said *N*-(substituted glycyl)-2-cyanopyrrolidine has the following formula (II) 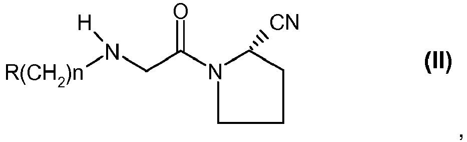 wherein
   R is substituted adamantyl; and
   n is 0-3.
10. The pharmaceutical composition according to any one of aspects 1 to 8, wherein said moisture-sensitive therapeutic compound is (S)-1-[(3-hydroxy-1-adamantyl)amino]acetyl-2-cyano-pyrrolidine.
11. The pharmaceutical composition of aspect 10, comprising between 50 mg and 100 mg of (S)-1-[(3-hydroxy-1-adamantyl)amino]acetyl-2-cyano-pyrrolidine.
12. A process for making granules comprising the steps of:
   (a) forming a mixture of a moisture-sensitive therapeutic compound with at least one hydrophobic melt component;
   (b) heating said mixture to a temperature substantially near the melting range of said hydrophobic melt component;
   (c) granulating said mixture under high shear to form said granules; and
   (d) cooling said granules to room temperature.
13. The process of aspect 12, wherein said mixture consists essentially of said moisture-sensitive therapeutic compound and said at least one hydrophobic melt component.
14. The process of aspect 12, wherein hydrophobic melt component is selected from the group consisting of esters, waxes, hydrocarbons, fatty alcohols, fatty acids, monoglycerides, diglycerides, triglycerides and mixtures thereof.
15. The process of aspect 12, wherein said moisture-sensitive therapeutic compound is a *N*-(substituted glycyl)-2-cyanopyrrolidine or a pharmaceutically acceptable salt thereof.
16. The process of aspect 15, wherein said *N*-(substituted glycyl)-2-cyanopyrrolidine is (S)-1-[(3-hydroxy-1-adamantyl)amino]acetyl-2-cyano-pyrrolidine.
17. A pharmaceutical composition comprising granules obtained by the process of aspect 12.
18. A pharmaceutical composition for oral administration comprising granules consisting essentially of a moisture-sensitive therapeutic compound an at least one hydrophobic melt component.
19. The pharmaceutical composition of aspect 18, wherein said granules are obtained by a melt granulation process.
20. The pharmaceutical composition of aspect 18, wherein said pharmaceutical composition releases at least about 50% of said moisture-sensitive therapeutic compound within 30 minutes after oral ingestion.
21. The pharmaceutical composition of Claim 18, wherein said pharmaceutical composition releases at least about 80% of said moisture-sensitive therapeutic compound within 30 minutes after oral ingestion.
22. The pharmaceutical composition of any one of aspects 17-22, wherein said moisture-sensitive therapeutic compound is (S)-1-[(3-hydroxy-1-adamantyl)amino]acetyl-2-cyanopyrrolidine.
23. The pharmaceutical composition of aspect 22, comprising between 50 mg and 100 mg of (S)-1-[(3-hydroxy-1-adamantyl)amino]acetyl-2-cyano-pyrrolidine.

The following examples are illustrative, but do not serve to limit the scope of the invention described herein. The examples are meant only to suggest a method of practicing the present invention.

Quantities of ingredients, represented by percentage by weight of the pharmaceutical composition, used in each example are set forth in the respective tables located after the respective descriptions.

### Example 1

### Solid Oral Dosage Form Prepared by Dry Blending

Compound I is first screened through a 25-mesh screen and 11.2 g is obtained. Compound I with 100 g of lactose are placed in a 1 quart V-blender and tumbled for 5 minutes. The mixture is removed, screened through a 25-mesh screen and returned to the V-blender. The talc, crospovidone and remaining lactose are then added to the V-blender which is tumbled for an additional 10 minutes. Separately, hydrogenated castor oil is passed through a 60-mesh screen. The hydrogenated castor oil is then added to the V-blender and tumbled for 5 minutes. The mixture is then compressed on a Manesty B3B tablet press using round, standard concave and beveled edge tooling. The tooling is polished before use to prevent filming. The obtained 150 mg tablets containing approximately 5 mg of Compound I are herein designated as "Sample 1". Sample 1 contains no particles of Compound I coated or substantially coated by the hydrogenated castor oil.

### Example 2

### Solid Oral Dosage Form Prepared from Melt Granulated Granules Using Hydrogenated Castor Oil

As a comparison against Sample 1, a solid oral dosage form is prepared from melt granulated Compound I. Compound I and a hydrophobic melt component, i.e., hydrogenated castor oil, are separately passed through a 25-mesh screen and 60-mesh screen, respectively. The ingredients are then added to a 1 L bowl of a Key International (Englishtown, NJ) Model KG5 high-shear granulator.

A heating mantel is wrapped around the bowl, and the rheostat is set at 80°C. The granulator is equipped with an impeller but no chopper. The impeller is turned on to allow mixing of the therapeutic compound and hydrophobic melt component.

After mixing, the granules are removed from the bowl and spread onto aluminum foil for cooling. The granules are subsequently passed through a mesh screen using a Frewitt oscillator.

The granules are then transferred to a V-blender with microcrystalline cellulose and crospovidone. The V-blender is tumbled for approximately 10 minutes. Afterwards, hydrogenated castor oil is added to the V-blender, and the mixture is allowed to tumble for an additional 5 minutes.

The mixture is then compressed on a Manesty B3B tablet press using round, standard concave and beveled edge tooling. The tooling is polished before use to prevent filming.

These tablets are designated herein as "Sample 2".

### Example 3

### Solid Oral Dosage Form Prepared from Melt Granulated Granules Using Stearic Acid

As a comparison against Samples 1 and 2, another solid oral dosage form is prepared from melt granulated granules of Compound I. The same process disclosed in Example 2 is used; however, stearic acid is substituted for the hydrogenated castor oil in its entirety. Thus, stearic acid is the hydrophobic melt component in the melt granulated granules and the lubricant in the tablet itself. These tablets are designated herein as "Sample 3".

Table 1 summarizes the compositions of the samples produced in Examples 1, 2 and 3.

**Table 1.**

| **Sample** | **Sample 1 (mg)** | **Sample 2 (mg)** | **Sample 3 (mg)** |
|---|---|---|---|
| Compound I | 3.7% | 3.7% | 3.7% |
| Hydrogenated castor oil as melt component | 0 | 13.3% | 0 |
| Stearic acid as melt component | 0 | 0 | 13.3% |
| Spray dried lactose as filler | 86.3% | 76.3% | 76.3% |
| Crospovidone as disintegrant | 4.7% | 4.7% | 4.7% |
| Talc as anti-adherent | 3.3% | 0 | 0 |
| Hydrogenated castor oil as lubricant | 2% | 2% | 0 |
| Stearic acid as lubricant | 0 | 0 | 2% |

Each of the samples are stored in induction sealed HDPE bottles for a period of 4 weeks at real-time aging conditions, i.e., 25°C / 75% RH and accelerated aging conditions, i.e., 40°C / 75% RH. After the 4-week period, the level of cyclic imidate, a degradation product of Compound I is measured. The results of the study are shown in Table 2 below.

**Table 2.**

| | **Time** | **Condition** | **Assay of Compound I (% label claim)** | **Cyclic imidate (%)** |
|---|---|---|---|---|
| **Sample 1** | Initial | | 92.4% | 0.12 |
| | 4 weeks | 25°C / 60% RH | 92.7% | 0.18 |
| | 4 weeks | 40°C / 75% RH | 93.0% | 0.81 |
| **Sample 2** | Initial | | 96.1% | 0.09 |
| | 4 weeks | 25°C / 60% RH | 92.3% | 0.12 |
| | 4 weeks | 40°C / 75% RH | 93.0% | 0.31 |
| **Sample 3** | Initial | | 86.8% | 0.09 |
| | 4 weeks | 25°C / 60% RH | 87.4% | 0.13 |
| | 4 weeks | 40°C / 75% RH | 84.8% | 0.34 |

After 4 weeks of accelerated aging, Sample 1 (the dry-blended tablets) experiences the greatest amount of degradation of the therapeutic compound. Thus, the therapeutic compound in Sample 1 has the greatest chemical instability and greatest susceptibility to degradation by moisture. The results for Samples 2 and 3 are comparable to each other and are better than that of Sample 1.

In addition to the stability testing, the three samples are tested for their dissolution profile. Almost complete release occurred after ten minutes for each of the three samples. The immediate-release profile of Sample 1 is expected since the tablets are processed by dry blending and compression. Surprisingly, however, even though the therapeutic compound is coated or substantially coated by a hydrophobic melt component in Samples 2 and 3, the granules still dissolved quickly and efficiently enough for an immediate-release profile.

### Example 4

### Solid Oral Dosage Form Prepared from Melt Granulated Compound I at 5 mg and 20 mg Dosage Strengths

Dosage strengths of 5 mg and 20 mg for Compound I are selected for the melt granulation process. These tablets, with a tablet weight of 125 mg and 250 mg, respectively, are manufactured using the process disclosed in Example 2. The ratio of Compound I and hydrogenated castor oil is about 1:4 in the melt granulated granules.

The resulting tablets are designated as follows: 125 mg tablets containing 5 mg of Compound I and lactose as "Sample 4A" and 250 mg tablets containing 20 mg of Compound I and lactose as "Sample 4B".

### Example 5

### Solid Oral Dosage Form Prepared from Melt Granulated Compound I at 50 mg and 100 mg Dosage Strengths

Dosage strengths of 50 mg and 100 mg for Compound I are additionally selected for the melt granulation process. These tablets, with a tablet weight of 250 mg, are manufactured using the same process disclosed in Example 2. The ratio of Compound I and hydrogenated castor oil is about 1:1 in the melt granulated granules.

The above tablets have been designated as follows: 250 mg tablets containing 50 mg of Compound I and lactose as "Sample 5A" and 250 mg tablets containing 100 mg of Compound I and lactose as "Sample 5B".

Table 3 summarizes the compositions of the samples produced in both Examples 4 and 5. The bolded ingredients constitute the melt granulated granules.

**Table 3.**

| **Sample (ratio of Compound I to hydrogenated castor oil)** | **Sample 4A (1:4)** | **Sample 4B (1:4)** | **Sample 5A (1:1)** | **Sample 5B (1:1)** |
|---|---|---|---|---|
| **Compound I** | **4.6% (w/w)** | **4.6%** | **11.5%** | **23.0%** |
| **Hydrogenated castor oil** | **16.0%** | **16.0%** | **10.0%** | **20.0%** |
| Lactose | 73.4% | 73.4% | 72.5% | 51.0% |
| Crospovidone | 5.0% | 5.0% | 5.0% | 5.0% |
| Hydrogenated castor oil | 1.0% | 1.0% | 1.0% | 1.0% |

### Example 6

### Solid Oral Dosage Form Prepared from Melt Granulated Compound I at 20 mg, 55 and100 mg Dosage Strengths with Microcrystalline Cellulose

Samples 4B, 5A and 5B are remade with the inclusion of microcrystalline cellulose, or AVICEL PH from FMC Corporation (Philadelphia, PA). Approximately half of the lactose in each of Samples 4B, 5A and 5B is replaced with microcrystalline cellulose. All other processing conditions are the same as described above. The Samples 4B, 5A and 5B with microcrystalline cellulose are renamed as Samples 6A, 6B and 6C, respectively.

Table 4 sets forth the compositions of Samples 6A, 6B and 6C.

**Table 4.**

| **Sample (ratio of Compound I to hydrogenated castor oil)** | **Sample 6A (1:4)** | **Sample 6B (1:1)** | **Sample 6C (1:1)** |
|---|---|---|---|
| **Compound I** | **4.6%** | **11.5%** | **23.0%** |
| **Hydrogenated castor oil** | **16.0%** | **10.0%** | **20.0%** |
| Lactose | 36.7% | 36.3% | 25.0% |
| Microcrystalline cellulose | 36.7% | 36.2% | 25.0% |
| Crospovidone | 5.0% | 5.0% | 5.0% |
| Hydrogenated castor Oil | 1.0% | 1.0% | 1.0% |

Table 5 shows the stability data for Samples 4A, 4B, 5A, 5B, 6A, 6B and 6C for 4 weeks at 25°C and 60% RH.

**Table 5.**

| | | **Time** | **Condition** | **Assay of Compound I (% label claim)** | **Cyclic imidate (%)** |
|---|---|---|---|---|---|
| **Sample 4A** | | | | | |
| | 5 mg of Compound I melt granulated | Initial | | 98.6% | 0.27% |
| | with hydrogenated castor oil and no microcrystalline cellulose | 4 weeks | 25°C / 60% RH | 99% | 0.25% |
| **Sample 4B** | | | | | |
| | 20 mg of Compound I melt | Initial | | 94.1% | 0.31% |
| | granulated with hydrogenated castor oil and no microcrystalline cellulose | 4 weeks | 25°C / 60% RH | 98.8% | 0.30% |
| **Sample 5A** | | | | | |
| | 50 mg of Compound I melt | Initial | | 101.0% | 0.11% |
| | granulated with hydrogenated castor oil and no microcrystalline cellulose | 4 weeks | 25°C / 60% RH | 102.7% | 0.21% |
| **Sample 5B** | | | | | |
| | 100 mg of Compound I melt | Initial | | 100.2% | 0.11% |
| | granulated with hydrogenated castor oil and no microcrystalline cellulose | 4 weeks | 25°C / 60% RH | 100.4% | 0.14% |
| **Sample 6A** | | | | | |
| | 20 mg of Compound I melt | Initial | | 95.3 | 0.28 |
| | granulated with hydrogenated castor oil and microcrystalline cellulose | 4 weeks | 25°C / 60% RH | 98.1 | 0.38 |
| **Sample 6B** | | | | | |
| | 50 mg of Compound I melt | Initial | | 101.1 | 0.15 |
| | granulated with hydrogenated castor oil and microcrystalline cellulose | 4 weeks | 25°C / 60% RH | 100.1 | 0.42 |
| **Sample 6C** | | | | | |
| | 100 mg of Compound I melt granulated with hydrogenated castor oil and microcrystalline cellulose | Initial | | 99.5 | 0.13 |
| | | 4 weeks | 25°C / 60% RH | 99.6 | 0.29 |

Table 6 shows the dissolution data for each of the Samples 4A, 4B, 5A, 5B, 6A and 6B at the 10 minute, 20 minute and 30 minute time points. Each of the samples are dissolved in 0.1 N hydrochloric acid and tested using paddles (Apparatus 2) at 37°C.

**Table 6.**

| | | **% Label Claim Dissolved** | | | |
|---|---|---|---|---|---|
| | | 0-time | 10 minutes | 20 minutes | 30 minutes |
| **Sample 4A** | | | | | |
| | 5 mg of Compound I melt granulated with hydrogenated castor oil and no microcrystalline cellulose | 0 | 73.7% | 87.9% | 91.3% |
| **Sample 4B** | | | | | |
| | 20 mg of Compound I melt granulated with hydrogenated castor oil and no microcrystalline cellulose | 0 | 79.1% | 86.0% | 89.4% |
| **Sample 5A** | | | | | |
| | 50 mg of Compound I melt granulated with hydrogenated castor oil and no microcrystalline cellulose | 0 | 100.0% | 101.7% | 101.7% |
| **Sample 5B** | | | | | |
| | 100 mg of Compound I melt granulated with hydrogenated castor oil and no microcrystalline cellulose | 0 | 34.8% | 79.4% | 100.9% |
| **Sample 6A** | | | | | |
| | 20 mg of Compound I melt granulated with hydrogenated castor oil and with microcrystalline cellulose | 0 | 72.1% | 78.5% | 81.9% |
| **Sample 6B** | | | | | |
| | 50 mg of Compound I melt granulated with hydrogenated castor oil and with microcrystalline cellulose | 0 | 88.2% | 93.7% | 94.4% |

For each of the Samples 4A, 4B, 5A, 5B, 6A and 6B, greater than 80% of Compound I as a percentage of label claim is dissolved in less than 30 minutes thereby showing the immediate-release characteristics of the formulations.

Another experiment (Examples 7 and 8) is conducted to determine the impact of an excipient with a high equilibrium content on Compound I in the presence of varying concentrations of the hydrophobic melt component. Once again, microcrystalline cellulose is used as the challenging excipient. The following samples are designated by the following ratio of x:y:z, in which "x" represents the concentration of Compound I, "y" represents the concentration of hydrogenated castor oil and "z" represents the concentration of microcrystalline cellulose.

### Example 7

### Melt Granulated Granules Challenged with Microcrystalline Cellulose

Using the process disclosed in Example 2, melt granulated granules of Compound I and hydrogenated castor oil are created to form five samples. All of these samples, except Sample 7A, contained melt granulated granules that are subsequently blended with microcrystalline cellulose in a ratio of 1:1 of Compound I:microcrystalline cellulose. However, the content of the hydrogenated castor oil varied within each sample. Table 7 shows the differences between each of the samples produced.

**Table 7.**

| **Sample (x:y:z)** | **Sample 7A (1:0:1)** | **Sample 7B (1:0.5:1)** | **Sample 7C (1:1:1)** | **Sample 7D (1:2:1)** | **Sample 7E (1:4:1)** |
|---|---|---|---|---|---|
| Compound I | 50% | 40% | 33.3% | 25% | 16.7% |
| Hydrogenated castor oil | 0% | 20% | 33.3% | 50% | 66.6% |
| Microcrystalline cellulose | 50% | 40% | 33.3% | 25% | 16.7% |

Table 8 shows the stability data for each of the samples in Example 7 at 40°C and 40°C and 75% RH. The stability data is of the tablets of each example induction sealed in high density polyethylene ("HDPE").

**Table 8.**

| **Ratio of x:y:z** | **Time** | **Condition** | **Assay (%)** | **CI (%)** |
|---|---|---|---|---|
| **Sample 7A** | Initial | | 98.89 | 0.06 |
| | 4 weeks | 40°C | 97.72 | 1.05 |
| | 4 weeks | 40°C / 75%RH | 93.79 | 3.04 |
| **Sample 7B** | Initial | | 99.64 | 0.07 |
| | 4 weeks | 40°C | 93.67 | 1.37 |
| | 4 weeks | 40°C / 75%RH | 95.99 | 1.80 |
| **Sample 7C** | Initial | | 98.16 | 0.07 |
| | 4 weeks | 40°C | 96.90 | 1.50 |
| | 4 weeks | 40°C / 75%RH | 96.65 | 1.56 |
| **Sample 7D** | Initial | | 101.54 | 0.07 |
| | 4 weeks | 40°C | 102.74 | 1.11 |
| | 4 weeks | 40°C / 75%RH | 101.79 | 1.45 |
| **Sample 7E** | Initial | | 95.22 | 0.07 |
| | 4 weeks | 40°C | 95.05 | 0.65 |
| | 4 weeks | 40°C / 75%RH | 92.87 | 1.13 |

From the results in Table 8, it is shown that the concentration of cyclic imidate resulting from the degradation of Compound I decreases as the level of the hydrophobic melt component, hydrogenated castor oil, increases. Surprisingly, the presence of hydrogenated castor oil reduces the sensitivity of Compound I to moisture even in the presence of a high equilibrium moisture content excipient, i.e., microcrystalline cellulose.

### Example 9

### Solid Oral Dosage Form with Compound IIC

A 10 mg tablet formulation of Compound IIC is produced by the following procedure. Compound IIC, microcrystalline cellulose, lactose, sodium starch glycolate are mixed in a 4-quart blender. The composition is mixed until the ingredients are thoroughly blended. Magnesium stearate is separately passed through a 25-mesh screen and subsequently added to the mixture in the blender. The dry-blended mixture is discharged from the blender and compressed on a rotary tablet press to form "Sample 9".

### Example 10

### Solid Oral Dosage Form Prepared from Melt Granulated Compound IIC

In comparison to Example 9, a 10 mg melt granulation formulation of Compound IIC is produced by the following procedure. Ten (10) mg of Compound IIC and 15 mg of stearyl alcohol are each screened through a 18-mesh screen into a Hobart mixer. A heating mantel is wrapped around the bowl, and the rheostat is set to 100. The mixing is continued until granules are formed in approximately 10-15 minutes of mixing time. The granules are then discharged onto aluminum foil and allowed to cool to room temperature. After cooling, the granules are passed through a 20-mesh screen. Half of the batch is removed for other purposes, with the remaining half blended with microcrystalline cellulose (128 g), lactose (64 g), sodium starch glycolate (12 g) and magnesium stearate (3 g) added to a Turbula blender. This mixture is mixed for 10 minutes. After mixing, the discharged mixture is compressed on a rotary tablet press. These tablets are designated herein as "Sample 10".

Samples 9 and 10 are each placed in induction sealed HDPE and tested for stability. The stability conditions included 3 weeks at 40°C and 75% RH; 6 weeks at 30°C and 60% RH; and 6 weeks at 40°C and 75%RH. Table 9 summarizes the percentage of total degradation product assayed after stability testing.

**Table 9.**

| | **Sample 9** | **Sample 10** |
|---|---|---|
| Initial | None detected | None detected |
| 3 weeks: 40°C / 75% RH | 0.08% | None detected |
| 6 weeks: 30°C / 60% RH | 0.06% | None detected |
| 6 weeks: 40°C / 75% RH | 0.23% | None detected |

From Table 9, it can be seen that the solid oral dosage form containing the melt granules (Example 10) has better chemical stability of the therapeutic compound than that of the dry-blended composition (Example 9).

The tablets of Example 10 are also tested for their dissolution in water using baskets at 50 rpm. After 30 minutes, over 90% of the therapeutic compound is released.

It is understood that while the present invention has been described in conjunction with the detailed description thereof that the foregoing description is intended to illustrate and not limit the scope of the invention, which is defined by the scope of the following claims. Other aspects, advantages and modifications are within the scope of the claims.

## Claims

1. DPP-IV inhibitor compound for use in treating non-insulin-dependent diabetes mellitus, wherein the compound is (S)-1-[(3-hydroxy-1-admantyl)amino]acetyl-2-cyanopyrrolidine of formula (IIC) 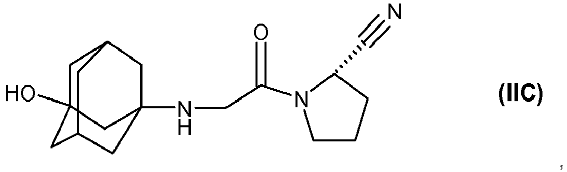 and optionally pharmaceutical salts thereof, wherein the dosage of the compound of formula (IIC) is between 50 and 100 mg per day and the application of the compound of formula (IIC) occurs twice a day.

2. DPP-IV inhibitor compound according to claim 1, wherein the dosage of the compound of formula (IIC) is 100 mg per day.

3. Pharmaceutical composition for the oral administration of a moisture-sensitive therapeutic compound, wherein the moisture-sensitive therapeutic compound is (S)-1-[(3-hydroxy-1-admantyl)amino]acetyl-2-cyano-pyrrolidine of formula (IIC) 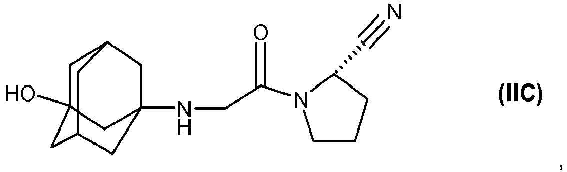 and optionally pharmaceutical salts thereof, wherein the composition comprises 50 or 100 mg of the compound of formula (IIC) and the application of the compound of formula (IIC) occurs twice a day.

4. Pharmaceutical composition according to claim 3, wherein the composition contains 50 mg of the compound of formula (IIC).

5. Pharmaceutical composition according to claim 3 or 4, wherein the composition is used for the treatment and/or prevention of non-insulin-dependent diabetes mellitus, arthritis, obesity, allograft transplantation and calcitonin osteoporosis.

6. Pharmaceutical composition for the oral administration of a moisture-sensitive therapeutic compound, wherein the moisture-sensitive therapeutic compound is (S)-1-[(3-hydroxy-1-admantyl)amino]acetyl-2-cyano-pyrrolidine of formula (IIC) 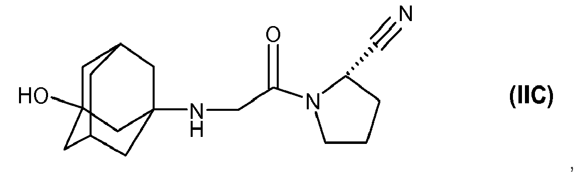 and optionally pharmaceutical salts thereof, wherein the dosage of the compound of formula (IIC) is between 50 and 100 mg per day and the application of the compound of formula (IIC) occurs twice a day.

7. Pharmaceutical composition according to claim 6, wherein the dosage of the compound of formula (IIC) is 100 mg per day.

8. Pharmaceutical composition according to claim 6 or 7, wherein the composition is used for the treatment and/or prevention of non-insulin-dependent diabetes mellitus, arthritis, obesity, allograft transplantation and calcitonin osteoporosis.
